# EUROPÄISCHE PATENTSCHRIFT

(11) **EP 0 337 056 B1**
(45) Veröffentlichungstag und Bekanntmachung des Hinweises auf die Patenterteilung: **29.03.1995**
(21) Anmeldenummer: 89100856.7
(22) Anmeldetag: 19.01.1989
(51) Int. Cl.: A61B 17/22

(54) **Einrichtung zum Orten und Zerstören von körperinneren Objekten mit Ultraschall**
Ultrasounds locating and destroying device for internal human body objects
Dispositif pour repérer et détruire des objets à l'intérieur du corps humain avec des ultrasons

(30) Priorität: 09.04.1988 DE 3811872
(43) Veröffentlichungstag der Anmeldung: 18.10.1989
(73) Patentinhaber: Richard Wolf GmbH, 75438 Knittlingen (DE)
(72) Erfinder: Wurster, Helmut, Dipl.-Ing., D-7519 Oberderdingen (DE); Krauss, Werner, Dipl.-Ing., D-7134 Knittlingen (DE); Vallon, Peter, D-7518 Bretten Gölshausen (DE)
(74) Vertreter: Wilcken, Thomas, Dipl.-Ing.

(56) Entgegenhaltungen:
- EP-A- 0 148 653
- EP-A- 0 257 199
- FR-A- 2 608 913

## Beschreibung

Die Erfindung betrifft eine Einrichtung zum Orten und Zerstören von körperinneren Objekten mit Ultraschall gemäß dem Oberbegriff des Anspruchs 1.

Derartige Einrichtungen sind seit längerem bekannt und auch im klinischen Einsatz.

Typische Beispiele derartiger Einrichtungen sind beispielsweise aus den DE-OS'en 33 19 871 und 27 22 252 sowie aus der DE-PS 31 19 295 bekannt.

Bei derartigen Einrichtungen werden fokussierende Ultraschallwellen auf ein im Körper befindliches Konkrement gerichtet, das infolgedessen zerstört wird. Diese Art der Zerstörung von Konkrementen stellt eine nichtoperative Methode zur Entfernung derselben dar. Nach Beaufschlagung des Konkrementes mit den fokussierten Stoßwellen befindet sich in dem betreffenden Organ, beispielsweise der Niere, lediglich ein feiner Grieß, der leicht auf natürliche Weise aus dem Körper herausgeschwemmt wird.

Vor Auslösung der Schußfolge relativ hochenergetischer Ultraschallwellen ist es erforderlich, die Lage des zu zerstörenden Konkrementes genau zu orten. Hierzu werden üblicherwise ein A- und/oder B-Scanner verwendet.

Ein Problem beim Einsatz der eingangs genannten Einrichtung besteht darin, daß benachbartes gesundes Gewebe durch den Einfluß der Ultraschallstoßwellen geschädigt werden kann, wenn der Fokus der Ultraschallsendeanordnung nicht auf das zu zerstörende Konkrement oder Gewebe fällt.

Die erforderliche Positionierungsgenauigkeit des Fokus im Verhältnis zum Stein hängt u.a. auch davon ab, nach welchem Prinzip die Ultraschallwellen erzeugt werden. Werden die Stoßwellen beispielsweise nach dem bekannten Unterwasser-Funkenentladungsprinzip erzeugt und über ellipsoide Reflektoren fokussiert, so ergeben sich typischerweise Brennpunktdurchmesser in der Größenordnung von etwa 10 mm.

Da dieser Fokusdurchmesser im Verhältnis zur üblichen Steingröße relativ groß ist, ist - infolge des relativ großen Fokusdurchmessers - bei diesem Verfahren keine hohe Positionierungsgenauigkeit erforderlich.

Werden hingegen die Ultraschallstoßwellen zur Zerstörung von Konkrementen im Körper durch besser fokussierende Stoßwellenquellen, beispielsweise durch piezoelektrische Wandler (vgl. DE-OS 33 19 871) erzeugt, so werden Fokusdurchmesser des Stoßwellendruckpulses mit einer Größenordnung von etwa 2 mm erreicht. Hieraus resultiert ein anderes Verhältnis zwischen der Steingröße einerseits und der Fokusgröße andererseits. Um einer Schädigung des das Konkrement umgebenden Gewebes infolge einer Auslösung einer Schußfolge von Stoßwellenimpulsen zu einem Zeitpunkt, wenn der Fokus nicht auf dem Stein liegt, entgegenzuwirken, wird eine exakte Positionierung des Fokus erforderlich.

Aber selbst wenn vor Auslösung einer Stoßwellenimpulsfolge der relativ engumgrenzte Fokus des Schallsenders auf dem zu zerstörenden Konkrement positioniert worden ist, kann nicht davon ausgegangen werden, daß der zu zerstörende Stein während der gesamten Therapiedauer seine Lage beibehält.

So verändert das zu zerstörende Konkrement, bzw. ein Stein seine Lage aufgrund periodisch wiederkehrender Körperfunktionen, insbesondere infolge des Ein- und Ausatmens des Patienten. Aber auch der erstmalige Beschuß des Konkrementes, z. B. in der Niere kann dessen Lage verändern.

Um in einem derartigen Fall, in dem das zu zerstörende Konkrement aus dem ursprünglich korrekt positionierten Fokus des Ultraschallsenders infolge einer Körperfunktion heraustritt, ein Auslösen von Schallimpulsfolgen zu vermeiden, sieht beispielsweise die DE-OS 36 21 935 eine Auslöseeinrichtung vor, die die Schußfreigabe in Abhängigkeit einer Körperfunktion steuert, und zwar in Abhängigkeit eines Vergleichs zwischen einem Schwellwert und einem Ist-Wert.

Aus EP 0 257 199 A1 ist eine Einrichtung bekannt, bei der eine elektronische Überwachung dahingehend vorgesehen wird, daß eine Auslösung von Stoßwellen nur dann erfolgen kann, wenn ein bestimmter Deckungsgrad zwischen dem Fokus des Therapiewandlers und dem Zielgebiet gegeben ist. Diese Einrichtung dient also im wesentlichen dazu, die regelmäßig wiederkehrende Verschiebung des Objektes, z.B. durch die Atemtätigkeit, bei der Behandlung zu berücksichtigen, insbesondere den Therapiewandler nur dann freizugeben, wenn die Überdeckung zwischen Wandlerfokus und Objekt gegeben ist.

Im übrigen ist es bekannt, Stoßwellen nicht nur zur Zerstörung von Konkrementen, sondern vielmehr auch zur Thromboisierung bspw. von Blutgefäßen oder auch zur direkten Zerstörung von krankhaftem Gewebe (Tumore) anzuwenden (vgl. DE-OS 35 44 344). Bei diesen Anwendungsfällen stellt sich im wesentlichen dasselbe Problem wie bei der Zerstörung von Konkrementen ein, nämlich daß dafür Sorge getragen werden muß, daß das benachbarte gesunde Gewebe durch die Ultraschallstoßwellen nicht beeinträchtigt wird.

Allen vorerwähnten körperfunktionsgesteuerten Auslöseeinrichtungen ist jedoch gemeinsam, daß sie von wiederkehrenden Körperfunktionen ausgehen und daher stets davon ausgehen, daß das Konkrement von sich aus wieder in den Fokusbereich wandert. Diese Einrichtungen ermöglichen zwar eine Schußfreigabe nur dann, wenn sich das Konkrement im Fokus befindet, sie geben jedoch keine Möglichkeit, das Konkrement in den Fokus zu bringen, wenn dies - aus welchen Gründen auch immer - aus einer wiederkehrenden Bewegung ausbricht. Dies kann beispielsweise durch den erstmaligen Beschuß erfolgen, wenn das Konkrement hierdurch seine Lage verändert oder wenn der Patient sich bewegt oder bewegt wird. Diese bekannten Einrichtungen sind daher nicht in der Lage, die Stoßwellenfreigabe in Abhängigkeit von Einzelereignissen, beispielsweise der vorerwähnten Verlagerung von körperinneren Objekten nach der erstmaligen Beschallung oder der Verlagerung des Patienten auf dem Behandlungstisch in geeigneter Weise zu berücksichtigen.

Ausgehend von diesem Stand der Technik liegt der Erfindung daher die Aufgabe zugrunde, eine Einrichtung zum Orten und Zerstören von körperinneren Objekten mit Ultraschall so auszubilden, daß auch derartige Einzelereignisse dahingehend berücksichtigt werden, daß das zu behandelnde Objekt schnell, zuverlässig und anwenderfreundlich geortet und mit dem Fokus des Therapiewandlers in Überdeckung gebracht werden kann.

Diese Aufgabe wird gelöst durch die Einrichtung mit den Merkmalen des Anspruchs 1.

Demnach wird vorgeschlagen, eine Einrichtung der gattungsgemäßen Art so auszubilden, daß ein ausgewähltes, das Objekt einschließendes Zielgebiet durch Verstellung des Wandlers automatisch abtastbar ist und daß der Impulsgenerator in Betrieb setzbar ist, wenn der Pegel des Ist-Wertes zumindest gleichgroß wie der Pegel des Soll-Wertes ist.

Das bildgebende System braucht hierbei kein ultraschalltechnisches Ortungssystem sein, es kann vielmehr auch ein Röntgen-Ortungssystem zum Einsatz kommen.

Die Verbindung des automatischen Abscannens des Zielgebietes durch die Verstellung des Wandlers mit der Schußfreigabe bei Überschreiten des gemessenen Helligkeitspegels eines vorgegebenen Soll-Wertes vereinigt in vorteilhafter Weise zum einen die Bedienungsfreundlichkeit der Einrichtung für das Personal beim exakten Auffinden des zu zerstörenden Objektes und beim Ausrichten des Fokus der Stoßwellen auf dieses Objekt und zum anderen den Sicherheitsaspekt hinsichtlich des Unterbindens einer Schußfreigabe bei nicht zweifelsfreier Koinzidenz der Lage des zu zerstörenden Objektes und des Fokus der Schallsenderanordnung, so daß mit größtmöglicher Sicherheit ausgeschlossen ist, daß gesundes Gewebe in der Nachbarschaft des zu zerstörenden Objektes durch die Stoßwellen geschädigt wird.

Besonders vorteilhaft ist die Ausbildung der erfindungsgemäßen Einrichtung, bei der der Soll-Wert so festgelegt ist, daß er unterhalb des Wertes liegt, der dem tatsächlichen Helligkeitswert des abgebildeten Objektes entspricht.

Da sich der Ist-Wert der Helligkeit durch Partikel- und/oder Steinstaubbildung in einem bestimmten Umfang reduzieren kann, und würde unter der Berücksichtigung des Sachverhaltes der Sollwert zu hoch angesetzt, könnte dies dazu führen, daß die Einrichtung durch ein zu schnelles Erreichen dieses Soll-Wertes den Wandler frühzeitig abschaltet, wodurch u.U. eine vollständige Steinzertrümmerung nicht möglich wäre.

Gemäß einer weiteren vorteilhaften Ausbildung der Einrichtung werden die Videosignale bildweise und die sich mit der Wandlerverstellung ändernden Koordinaten der Zielmarkenposition auf einen Steuerkreis mit einem Speicher übertragen, die der Lage der Zielmarke zugeordneten Bildsignale aus dem Speicher ausgelesen,diese Bildsignale als Ist-Werte einem Eingang eines Vergleichskreises zugeführt, an dessen anderem Eingang ein dem Soll-Wert entsprechendes Signal ansteht, wobei an den Ausgang des den Vergleich zwischen Ist-Werten und Soll-Wert durchführenden Vergleichskreises eine Steuerung zur Sperrung oder Freigabe des Impulsgeneratorbetriebes angeschlossen ist.

Die Verstellbewegungen und Positionen des Wandlers sind vorteilhaft mit Positionsgebern erfaßbar, die mit den motorischen Wandlerantrieben zusammenarbeiten, und die Positionsgeber sind an die Eingänge der erwähnten Steuerung angeschlossen.

In vorteilhafter Weiterbildung sind die jeweils vorgegebenen Verstellbewegungen des Wandlers programmiert.

Hierbei kann der Wandler zum automatischen Abscannen in einer durch eine vorgegebene Zielfläche festgelegten X,Y-Ebene verstellbar sein oder ein automatisch abzuscannendes räumliches Zielgebiet vorgegeben werden, wobei der Wandler dann auf räumlichen Koordinaten X,Y und Z verstellbar ist.

Das bildgebende System weist vorteilhafter Weise zumindest einen B-Scanner auf, der mit dem Wandler eine bauliche Einheit bildet und relativ zu diesem verdrehbar und axial verstellbar ist.

Alternativ hierzu ist eine Ausführungsform der Einrichtung möglich, bei der das bildgebende System zumindest einen B-Scanner aufweist und der Wandler relativ zum feststehenden B-Scanner verstellbar ist.

Hierbei ist der Wandler zum automatischen Abscannen vorzugsweise um zwei Achsen schwenkbar gelagert, so daß durch Abstimmung der Winkelgeschwindigkeiten der Schwenkbewegungen kreisförmige oder elliptische Bewegungsbahnen des Wandlerfokusses erzeugt werden können.

Ganz generell kann der Wandler vorzugsweise mit seinem Fokus zur Ortung des Objektes im Zielgebiet auf beliebigen sich in Richtung auf ein gemeinsames Zentrum bewegenden Raumbahnen um die Wandlersymmetrieachse verschwenkbar sein.

Da eine Verstellung des Wandlers nicht nur mittels einer oder mehrerer Schwenkachsen erfolgen kann, sondern auch durch teleskopartig verstellbare Lager,kann die erfindungsgemäße Einrichtung auch so ausgebildet sein, daß mindestens drei den Wandler aufnehmende Lager zur Durchführung einer beliebigen Schwenk- und/oder Taumelbewegung in axialer Richtung verstellbar sind.

Eine weitere vorteilhafte Weiterbildung der Einrichtung ist dann gegeben, wenn die Zielmarke unabhängig von der Wandlerverstellung auf dem Monitor ständig den Fokus des Wandlers repräsentiert.

Die Erfindung wird anhand von Ausführungsbeispielen gemäß der Zeichnungen näher dargestellt. Hierbei zeigt:
- Figur 1: ein Blockschaltbild der erfindungsgemäßen Vorrichtung,
- Figur 2: ein Blockschaltbild der Ansteuerung für den Motor, mit Hilfe dessen der Wandler das Zielgebiet abscannt, sowie eine schematische Darstellung des abgescannten Zielgebietes, und zwar zwei- und dreidimensional dargestellt,
- Figur 3: den schematischen Aufbau eines Antriebes zum Kippen des Wandlers, bei dem der Ultraschallscanner stehenbleibt,
- Figur 4: das Blockschaltbild für eine Ansteuerung der in Figur 3 dargestellten Wandleranordnung.

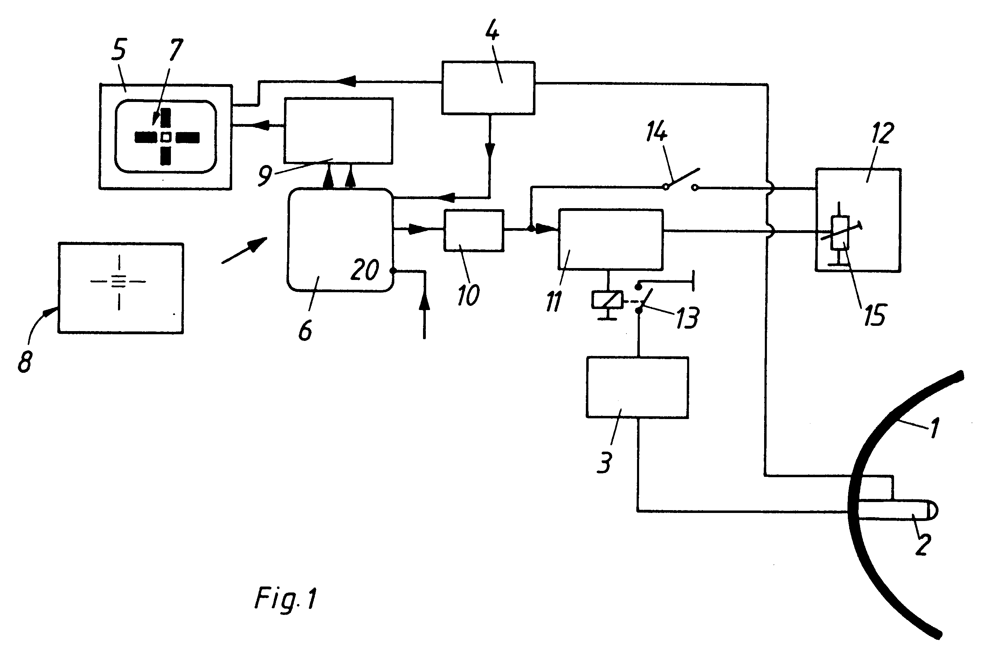

Figur 1 zeigt schematisch einen Ultraschallwandler 1, der fest mit einem Ultraschallscanner 2 verbunden ist. Der Ultraschallwandler 1 ist bei Vorliegen eines Freigabesignals - wie unten näher erläutert - von einem Impulsgenerator 3 ansteuerbar.

Das vom Ultraschallscanner 2 empfangene Signal wird in seiner Helligkeit von einem Helligkeitsmesser 4 erfaßt und ist auf einem diesem Helligkeitsmesser 4 nachgeschaltete, Monitor 5 abbildbar. Daneben sind die im Helligkeitsmesser 4 erfaßten Helligkeitswerte des Ultraschallscannersignals an einen Matrixspeicher 6 geführt und in diesen einspeicherbar.

Auf dem Monitor 5 ist eine Zielmarke 7 abbildbar, deren Lage auf dem dargestellten zweidimensionalen Bild auf dem Bildschirm der Lage einer virtuellen Zielmarke 8 im Matrixspeicher 6 entspricht. Die Marke 8 im Matrixspeicher 6 dient der Adressierung eines zweidimensionalen Feldes von im Matrixspeicher 6 abgespeicherten Helligkeitswerten des Ultraschallscannersignals.

Über eine Zielmarkensteuerung 9 zwischen dem Matrixspeicher 6 und dem Monitor 5 ist gewährleistet, daß die Zielmarke 7 auf dem Bildschirm exakt dasselbe Feld umgrenzt, welches von der virtuellen Zielmarke 8 im Matrixspeicher 6 adressiert ist.

Die Lage der virtuellen Zielmarke 8 im Matrixspeicher 6 und damit mittelbar die Zielmarke 7 auf dem Monitor ist einstellbar über ein entsprechendes Signal, welches dem Matrixspeicher 6 über einen Eingang 20 zugeführt wird.(vgl. unten).

Der Inhalt des Matrixspeichers 6 in dem von der virtuellen Zielmarke umgrenzten Feld wird einem Mittelwertbildner 10 zugeführt, der einen Mittelwert der Helligkeiten allermatrixpunkte , welche in dem umgrenzten Zielmarkenfeld liegen, ermittelt. Der ermittelte Wert wird einem Vergleicher 11 zugeführt, an dem gleichfalls ein zuvor festgelegter Sollwert aus einem Sollwertspeicher 12 anliegt.

Der Sollwert wird in der Weise ermittelt, daß nach Betätigung eines Rücksetzschalters 14 der Istwert des gemittelten Helligkeitswertes der von der Zielmarke 8 im Matrixspeicher 6 bei einer Zielmarkenlage, in der das zu zerstörende Objekt im Zielgebiet liegt, wodurch sich die maximalen Helligkeitswerte ergeben, erfaßten Bildpunkte im Sollwertspeicher 12 abgespeichert werden. Wenn der momentan am Vergleicher 11 anstehende Istwert des Helligkeitspegels zumindest gleichgroß ist wie der Pegel des Sollwertes aus dem Sollwertspeicher 12, wird ein mit dem Impulsgenerator 3 in geeigneter Weise verbundener Freigabeschalter 13 betätigt, das heißt, daß die Einrichtung in diesem Betriebszustand Stoßwellen auf das im Fokus befindliche, zu zerstörende Objekt abgeben kann.

Aufgrund der beschriebenen, dem Sicherheitsaspekt der der Erfindung zugrundeliegenden Aufgabe Rechnung tragenden Schaltung ist gewährleistet,daß eine Freigabe für die Auslösung einer Ultraschallstoßwelle durch den Wandler 1 stets nur dann gegeben wird, wenn das zu zerstörende Objekt im Zielgebiet, das heißt im Fokusbereich, liegt.

Der im Sollwertspeicher 12 abgespeicherte Sollwert ist vorzugsweise über ein Einstellglied 15 einstellbar, derart, daß er unterhalb des Wertes liegt, der den tatsächlichen Helligkeitswert des abgebildeten Objektes, wenn dieses im Zielgebiet liegt, entspricht. Vorzugsweise wird der Sollwert zu dem 0.8fachen des tatsächlichen Helligkeitswertes gewählt. Dies ist dann von Relevanz, wenn sich der Istwert des Helligkeitspegels durch Partikel- und Steinstaubbildung nach den ersten applizierten Stoßwellen in einem bestimmten Umfang reduziert. Würde in einem solchen Fall der Sollwert zu hoch angesetzt werden, beispielsweise zu 100% des maximalen Helligkeitswertes vor erfolgter Applikation von Stoßwellen, könnte dies dazu führen, daß die Einrichtung durch ein zu schnelles Erreichen dieses Sollwertes den Wandler frühzeitig abschaltet, wodurch eine weitere Applikation von Stoßwellen auf das zu zerstörende Objekt nicht möglich wäre.

Ein weiterer Aspekt der Erfindung ist das automatische Abscannen des Zielgebietes durch Verstellung des Wandlers, wie nachfolgend beschrieben wird.

Die Verstellung des Hauptwandlers 1 mit dem feststehenden Ultraschallscanner 2 erfolgt durch Motoren MₓM_{y}M_{z}, die ihrerseits von einem Logikmotorverstärker 16 angesteuert werden. Der Logikmotorverstärker 16 erhält seinerseits Signale von einem Rechner 17, der seinerseits Eingabesignale von einer Eingabeeinheit 18 erhält.

In die Eingabeeinheit 18 werden die Koordinaten X₁,X₂,Y₁,Y₂, Z₁ und Z₂ sowie ΔX,ΔY und ΔZ eingegeben. Dabei geben die indizierten Koordinaten die Koordinaten eines Volumenelementes an, innerhalb dessen sich das zu zerstörende Objekt befindet, und die Δ - Werte die entsprechenden Schrittweiten während des Abscanvorganges.

Die eingegebenen Werte werden einem im Rechner 17 gespeicherten Programm zugeführt, welches daraufhin den Verlauf der Verstellbewegungen des Wandlers festlegt. Der Rechner steuert dementsprechend den Logikmotorverstärker 16.

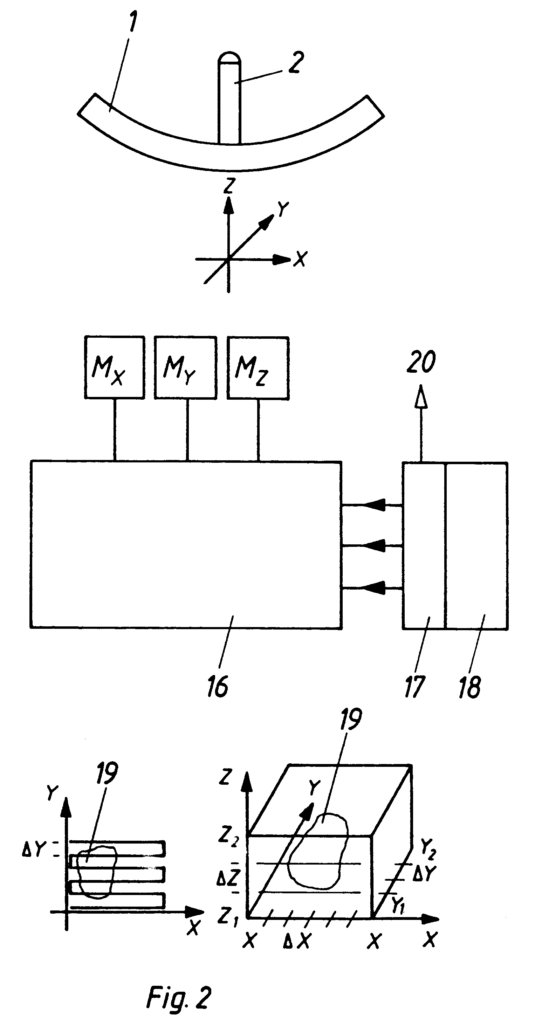

In Figur 2 unten ist ein beispielhafter Verlauf des automatischen Abscannens eines zu zerstörenden Objektes 19 schematisch dargestellt, wobei die linke untere Darstellung lediglich eine zweidimensionale Verstellbewegung des Wandlers in X,Y-Richtung andeutet, wohingegen die rechte untere Darstellung auch ein Abscannen in Z-Richtung beinhaltet.

Da die Fokuslänge zumeist etwa eine Größenordnung von 1O mm einnimmt und dies der Größenordnung der meisten zu zerstörenden Objekte entspricht, reicht in den meisten Fällen ein zweidimensionales Abscannen des fraglichen Objektes aus.

Im übrigen werden die in die Eingabeeinheit 18 eingegebenen Koordinaten über den Rechner 17 dem Matrixspeicher 6 (Fig. 1) über dessen Eingang 20 zugeführt.

Damit wird gewährleistet, daß sich zum einen die Lage der Zielmarke 7 auf dem Monitor sowie der virtuellen Marke 8 im Matrixspeicher 6 entsprechend den Verstellbewegungen des Wandlers ändert und zum anderen, daß die Zielmarken 7,8 stets den Fokusbereich des Wandlers repräsentieren.

Bewegt sich bei der Anwendung der beschriebenen Einrichtung das zu zerstörende Objekt aus dem Zielgebiet hinaus, wird die Freigabe für die Stoßwellen automatisch gestoppt, solange,bis durch das automatisch erfolgende Abscannen des Zielgebietes die Lage des Objektes ermittelt worden ist, so daß daraufhin aufgrund der erfolgenden Helligkeitspegelerfassung des Zielgebietes eine erneute Freigabe für die Applikation von Stoßwellen erfolgen kann.

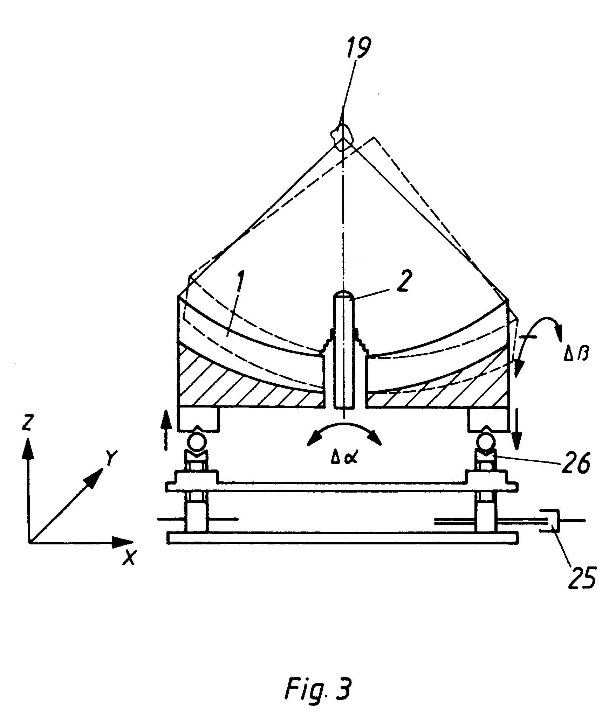

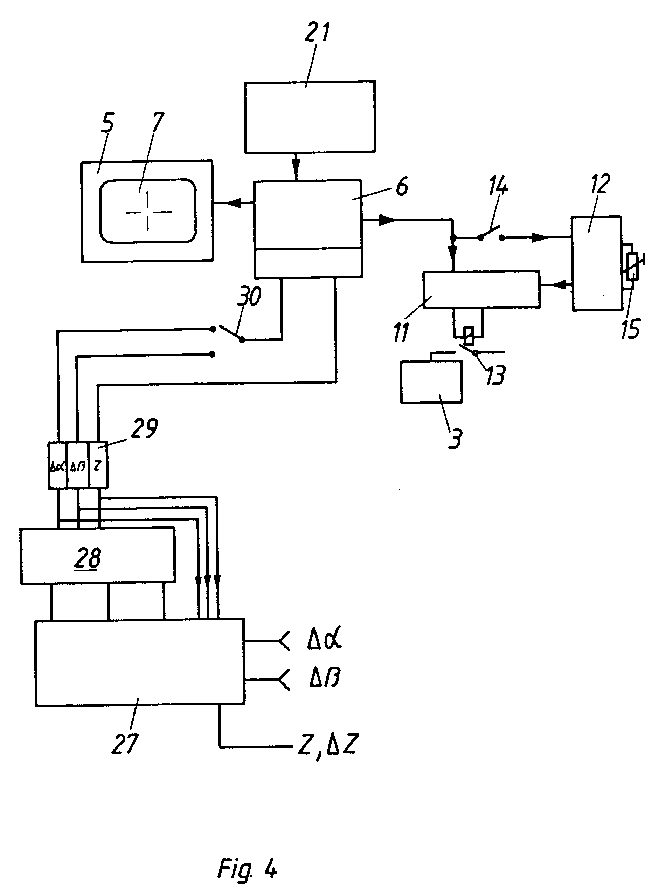

Eine weitere Ausführungsform der erfindungsgemäßen Einrichtung im Hinblick auf das automatische Abscannen des Zielgebietes des zu zerstörenden Objektes soll anhand der Figuren 3 und 4 erläutert werden.

Figur 3 zeigt einen Ultraschallwandler 1 mit einem Ultraschallscanner 2 , der in diesem Ausführungsbeispiel jedoch stehenbleibt bei einer Verstellbewegung des Wandlers 1. In diesem Ausführungsbeispiel ist die Wandlerkalotte um einen Mittelpunkt in den Achsen X und Y jeweils um einen kleinen Winkel Δα bzw. Δβ schwenkbar gelagert.

Hierzu ist die Wandlerkalotte bspw.mittels vier Gewindestangen 26 auf- und abbewegbar, wozu diese mit einem Elektromotor oder dgl. angetrieben werden.

Dieselbe Art der Lagerung der Kalotte ist im übrigen um die andere Achse vorgesehen, so daß sich durch die Überlagerung der Bewegungen ein Kreis oder eine Ellipse ergibt. Die ausgeführte Bewegung des Wandlers 1 wird moduliert über der Zeit, so daß stets die Ellipse von Null bis zum Maximalwert, der im übrigen einstellbar ist,spiralförmig durchlaufen wird.

Für die Freigabe eines Stoßwellenimpulses wird die Schaltung gemäß Fig. 1 verwendet. Dazu müssen die Zielmarken 7,8 der vom Wandler ausgeführten Pendelbewegung entsprechend nachgeführt werden.

Die Eingabe der Verstellwinkel Δ α , Δ β , sowie der Koordinate Z bzw. Δ Z erfolgt über die Steuerung 27, welche ihrerseits Motoren 28 antreibt. Die momentanen, vom Wandler 1 eingenommenen Koordinaten werden von Positionsgebern 25 erfaßt und über Geber 29 an den Matrixspeicher 6 geführt. Der Schalter 3O dient zum Umschalten der Ultraschallebene entsprechend der Bildlage des Ultraschallscanners. Dabei korreliert eine Verschwenkbewegung um den Winkel Δ α mit einer Darstellung in der X-Y-Ebene, und entsprechend um den Winkel Δ β mit einer Darstellung in der Y-Z-Ebene.

Wie bereits erwähnt, entspricht die elektronische Schaltung zur Steuerung der Freigabe des Stoßwellenimpulses der Schaltung aus Figur 1. In Figur 4 ist lediglich mit dem Bezugszeichen 21 ein Ultraschall-Bildgerät (beispielsweise B-Scanner) als ein schematisch dargestelltes Bauelement dargestellt.

Im übrigen braucht die Wandlerkalotte gemäß dem vorstehend beschriebenen Ausführungsbeispiel nicht auf Gewindestangen 26 gelagert sein, vielmehr ist es auch möglich, die Kalotte zur Durchführung einer beliebigen Schwenk- und/oder Taumelbewegung auf zumindest drei Lager zu lagern, welche in diesem Fall axial verstellbar sein müssen.

## Patentansprüche

1. Einrichtung zum Orten und Zerstören von körperinneren Objekten (19) mit Ultraschall, bestehend aus einem von einem Impulsgenerator (3) betriebenen Wandler (1) zur Erzeugung von fokussierten Stoßwellen, die über ein Koppelmedium auf den Körper des Patienten übertragbar sind, und aus einem mit dem Wandler (1) verbundenen bildgebenden Ortungssystem (2, 5) mit wenigstens einem Monitor (5) als Sichtgerät, auf dessen Bildschirm das Objekt (19) und eine Zielmarke (7) darstellbar sind, die durch eine Relativbewegung zwischen Patient und Wandler (1) in Überdeckung bringbar sind, um den Wandlerfokus auf das Objekt (19) auszurichten und den Betrieb des Impulsgenerators (3) freizugeben, wobei ein dem Helligkeitswert des abgebildeten Objektes (19) entsprechender, aus dem Videosignal des bildgebenden Systems (2, 5) gewonnener Signalpegel als Soll-Wert festlegbar ist und die hierbei im bildgebenden System (2, 5) anfallenden Videosignale als Ist-Werte mit dem Soll-Wert vergleichbar sind, dadurch gekennzeichnet, daß ein ausgewähltes, das Objekt (19) einschließendes Zielgebiet durch Verstellung des Wandlers (1) automatisch abtastbar ist und daß der Impulsgenerator (3) in Betrieb setzbar ist, wenn der Pegel des Ist-Wertes zumindest gleichgroß wie der Pegel des Soll-Wertes ist.

2. Einrichtung nach Anspruch 1, dadurch gekennzeichnet, daß der Soll-Wert so festgelegt ist, daß er unterhalb des Wertes liegt, der dem tatsächlichen Helligkeitswert des abgebildeten Objektes (19) entspricht.

3. Einrichtung nach einem der Ansprüche 1 und 2, dadurch gekennzeichnet, daß die Videosignale bildweise und die sich mit der Wandlerverstellung ändernden Koordinaten der Zielmarkenposition auf einen Steuerkreis mit einem Speicher (6) übertragbar sind, daß aus dem Speicher (6) die der Lage der Zielmarke (7) zugeordneten Bildsignale auslesbar sind, daß diese Bildsignale als Ist-Werte dem Eingang eines Vergleichskreises (11) zuführbar sind, an dessen anderem Eingang ein dem Soll-Wert entsprechendes Signal ansteht, und daß an den Ausgang des den Vergleich zwischen Ist-Werten und Soll-Wert durchführenden Vergleichskreises (11) eine Steuerung (13) zur Sperrung oder Freigabe des Impulsgeneratorbetriebes angeschlossen ist.

4. Einrichtung nach einem der Ansprüche 1 bis 3, dadurch gekennzeichnet, daß die Verstellbewegungen und Positionen des Wandlers (1) mit Positionsgebern (25) erfaßbar sind, die mit den motorischen Wandlerantrieben zusammenarbeiten, und daß die Positionsgeber (25) an die Eingänge der erwähnten Steuerung angeschlossen sind.

5. Einrichtung nach einem der Ansprüche 1 bis 4, dadurch gekennzeichnet, daß die jeweils vorgegebenen Verstellbewegungen des Wandlers (1) programmiert sind.

6. Einrichtung nach einem der Ansprüche 1 bis 5, dadurch gekennzeichnet, daß der Wandler (1) zum automatischen Abscannen in einer durch eine vorgegebene Zielfläche festgelegten X,Y-Ebene verstellbar ist.

7. Einrichtung nach einem der Ansprüche 1 bis 5, dadurch gekennzeichnet, daß ein automatisch abzuscannendes räumliches Zielgebiet vorgebbar und der Wandler (1) auf räumlichen Koordinanten X,Y und Z verstellbar ist.

8. Einrichtung nach einem der Ansprüche 1 bis 7, dadurch gekennzeichnet, daß das bildgebende System zumindest einen B-Scanner (2) aufweist, der mit dem Wandler (1) eine bauliche Einheit bildet und relativ zu diesem verdrehbar und axial verstellbar ist.

9. Einrichtung nach einem der Ansprüche 1 bis 7, dadurch gekennzeichnet, daß das bildgebende System zumindest einen B-Scanner (2) aufweist und daß der Wandler (1) relativ zum feststehenden B-Scanner (2) verstellbar ist.

10. Einrichtung nach Anspruch 9, dadurch gekennzeichnet, daß der Wandler (1) zum automatischen Abscannen um zwei Achsen verschwenkbar gelagert ist und daß durch Abstimmung der Winkelgeschwindigkeiten der Schwenkbewegungen kreisförmige oder elliptische Bewegungsbahnen des Wandlerfokusses erzeugbar sind.

11. Einrichtung nach einem der Ansprüche 1 bis 10, dadurch gekennzeichnet, daß der Wandler (1) mit seinem Fokus zur Ortung des Objektes (19) im Zielgebiet auf beliebigen sich in Richtung auf ein gemeinsames Zentrum bewegenden Raumbahnen um die Wandlersymmetrieachse verschwenkbar ist.

12. Einrichtung nach einem der Ansprüche 1 bis 11, dadurch gekennzeichnet, daß mindestens drei den Wandler (1) aufnehmende Lager zur Durchführung einer beliebigen Schwenk- und/oder Taumelbewegung in axialer Richtung verstellbar sind.

13. Einrichtung nach einem der Ansprüche 1 bis 12, dadurch gekennzeichnet, daß die auf dem Monitor (5) dargestellte Zielmarke (7) unabhängig von der Wandlerverstellung immer den Fokus des Wandlers (1) repräsentiert.

## Claims

1. A device for locating and destroying with ultrasonics objects (19) inside the body, consisting of a transducer (1), operated by an impulse generator (3), for the production of focussed shock waves, which are able to be transmitted via a coupling medium to the body of the patient, and of an image-emitting locating system (2,5), connected with the transducer (1), with at least one monitor (5) as visual apparatus, on the screen of which the object (19) and a target mark (7) are able to be illustrated, which are able to be brought to overlap by a relative movement between patient and transducer (1), in order to align the transducer focus onto the object (19) and to release the operation of the impulse generator (3), in which a signal level, corresponding to the brightness value of the pictured object (19), obtained from the video signal of the image-emitting system (2,5), is able to be established as nominal value, and the video signals occurring in this connection in the image-emitting system (2,5) are able to be compared as actual values with the nominal value, characterised in that a selected target area, including the object (19), is able to be scanned automatically by adjusting the transducer (1) and that the impulse generator (3) is able to be set in operation when the level of the actual value is at least equal to the level of the nominal value.

2. A device according to Claim 1, characterised in that the nominal value is established such that it lies beneath the value which corresponds to the actual brightness value of the pictured object (19).

3. A device according to one of Claims 1 and 2, characterised in that the video signals are able to be transmitted by image and the coordinates of the target mark position, changing with the transducer adjustment, are able to be transmitted to a control circuit with a memory (6), that the image signals associated with the position of the target mark (7) are able to be read from the memory (6), that these image signals are able to be supplied as actual values to the input of a comparison circuit (11), at the other input of which a signal waits which corresponds to the nominal value, and that at the ouput of the comparison circuit (11) carrying out the comparison between actual values and nominal value, a control arrangement (13) is connected to block or release the impulse generator operation.

4. A device according to one of Claims 1 to 3, characterised in that the adjustment movements and positions of the transducer (1) are able to be picked up by position indicators (25), which cooperate with the motor transducer drives, and that the position indicators (25) are connected to the inputs of the said control arrangement.

5. A device according to one of Claims 1 to 4, characterised in that the respectively given adjustment movements of the transducer (1) are programmed.

6. A device according to one of Claims 1 to 5, characterised in that the transducer (1) is able to be adjusted for automatic scanning in an X,Y plane set by a given target area.

7. A device according to one of Claims 1 to 5, characterised in that a three-dimensional target area, which is to be scanned automatically, is able to be preset and the transducer (1) is able to be adjusted to three-dimensional coordinates X, Y and Z.

8. A device according to one of Claims 1 to 7, characterised in that the image-emitting system has at least one B-scanner (2), which forms a structural unit with the transducer (1) and is rotatable in relation thereto, and axially adjustable.

9. A device according to one of Claims 1 to 7, characterised in that the image-emitting system has at least one B-scanner (2) and that the transducer (1) is adjustable relative to the fixed B-scanner (2).

10. A device according to Claim 9, characterised in that the transducer (1) is mounted so as to be pivotable about two axes for automatic scanning and that by adjusting the angular velocities of the pivotal movements, circular or elliptical paths of movement of the transducer focus are able to be produced.

11. A device according to one of Claims 1 to 10, characterised in that the transducer (1) is pivotable about the symmetry axis of the transducer with its focus, to locate the object (19) in the target area, on any spatial paths moving in the direction towards a common centre.

12. A device according to one of Claims 1 to 11, characterised in that at least three bearings holding the transducer (1) are adjustable, to carry out any desired pivotal- and/or tumbler movement in axial direction.

13. A device according to one of Claims 1 to 12, characterised in that the target mark (7) illustrated on the monitor (5) always represents the focus of the transducer (1) independently of the transducer adjustment.

## Revendications

1. Mécanisme pour la détection et la destruction d'objets (19) à l'intérieur du corps à l'aide d'ultrasons, constitué par un transducteur (1) entraîné par un générateur d'impulsions (3) pour générer des ondes de choc focalisées qui peuvent être transmises au corps du patient à l'intervention d'un milieu d'accouplement, et par un système de détection (2, 5) formateur d'images relié au transducteur (1) et comprenant au moins un moniteur (5) comme unité d'affichage sur l'écran de laquelle on peut représenter l'objet (19) et un repère (7) qui peuvent être amenés en superposition à l'intervention d'un mouvement relatif entre le patient et le transducteur (1) pour aligner le foyer du transducteur sur l'objet (19) et pour libérer l'entraînement du générateur d'impulsions (3), dans lequel un niveau de signal correspondant à la luminosité de l'objet affiché (19) et obtenu à partir du signal vidéo du système (2, 5) formateur d'images peut être déterminé comme valeur de consigne, les signaux vidéo que l'on obtient en l'occurrence dans le système (2, 5) formateur d'images pouvant être comparés sous forme de valeurs réelles à la valeur de consigne, caractérisé en ce qu'un domaine cible sélectionné englobant l'objet (19) peut être balayé automatiquement en déplaçant le transducteur (1), et en ce que le générateur d'impulsions (3) peut être mis en service lorsque le niveau de la valeur réelle est au moins aussi élevé que le niveau de la valeur de consigne.

2. Mécanisme selon la revendication 1, caractérisé en ce qu'on détermine la valeur de consigne de telle sorte qu'elle se situe en dessous de la valeur qui correspond à la luminosité effective de l'objet affiché (19).

3. Mécanisme selon l'une quelconque des revendications 1 et 2, caractérisé en ce que les signaux vidéo peuvent être transmis en forme d'images et les coordonnées de la position de repère, qui se modifient avec le déplacement du transducteur, peuvent être transmises à un circuit de commande comprenant une mémoire (6), en ce qu'on peut lire, à partir de la mémoire (6), les signaux d'images attribués à la position du repère (7), en ce que ces signaux d'images peuvent être acheminés sous forme de valeurs réelles à l'entrée d'un circuit de comparaison (11) à l'autre entrée duquel apparaît un signal correspondant à la valeur de consigne, et en ce que, à la sortie du circuit de comparaison (11) effectuant la comparaison entre les valeurs réelles et la valeur de consigne, est raccordée une commande (13) pour arrêter ou libérer l'entraînement du générateur d'impulsions.

4. Mécanisme selon l'une quelconque des revendications 1 à 3, caractérisé en ce que les mouvements de déplacement et les positions du transducteur (1) peuvent être enregistrés à l'aide d'indicateurs de position (25) qui coopèrent avec l'entraînement mécanique du transducteur, et en ce que les indicateurs de position (25) sont raccordés aux sorties de la commande mentionnée.

5. Mécanisme selon l'une quelconque des revendications 1 à 4, caractérisé en ce que les mouvements de déplacement du transducteur (1) respectivement prédéfinis sont programmes.

6. Mécanisme selon l'une quelconque des revendications 1 à 5, caractérisé en ce que le transducteur (1) peut se déplacer, pour le balayage automatique, dans un plan X, Y établi par une surface cible prédéfinie.

7. Mécanisme selon l'une quelconque des revendications 1 à 5, caractérisé en ce qu'on peut prédéfinir un domaine spatial qui doit faire l'objet d'un balayage automatique, le transducteur (1) pouvant se déplacer en fonction de coordonnées spatiales X, Y et Z.

8. Mécanisme selon l'une quelconque des revendications 1 à 7, caractérisé en ce que le système formateur d'images présente au moins un scanner (2) de type B qui forme, avec le transducteur (1), une unité de construction et qui peut effectuer une rotation par rapport à ce dernier, tout en pouvant se déplacer en direction axiale.

9. Mécanisme selon l'une quelconque des revendications 1 à 7, caractérisé en ce que le système formateur d'images présente au moins un scanner (2) de type B et en ce que le transducteur (1) peut se déplacer par rapport au scanner (2) de type B disposé à demeure.

10. Mécanisme selon la revendication 9, caractérisé en ce que le transducteur (1) pour le balayage automatique est monté en pivotement autour de deux axes et en ce qu'on peut obtenir des trajectoires de mouvement circulaires ou elliptiques du foyer du transducteur par accord aux vitesses angulaires des mouvements de pivotement.

11. Mécanisme selon l'une quelconque des revendications 1 à 10, caractérisé en ce que le transducteur (1), à des fins de détection de l'objet (19) dans le domaine cible, peut pivoter autour de l'axe de symétrie du transducteur, sur n'importe quelle trajectoire spatiale se déplaçant en direction d'un centre commun.

12. Mécanisme selon l'une quelconque des revendications 1 à 11, caractérisé en ce qu'au moins trois paliers dans lesquels viennent se loger le transducteur (1) peuvent se déplacer en direction axiale pour effectuer n'importe quel mouvement de pivotement et/ou de basculement.

13. Mécanisme selon l'une quelconque des revendications 1 à 12, caractérisé en ce que le repère (7) représenté sur le moniteur (5) indique toujours le foyer du transducteur (1) indépendamment du déplacement du transducteur.
